# EUROPÄISCHE PATENTANMELDUNG

(11) **EP 1 029 911 A1**
(43) Veröffentlichungstag der Anmeldung: **23.08.2000**
(21) Anmeldenummer: 00102608.7
(22) Anmeldetag: 08.02.2000
(51) Int. Cl.: C11D 3/37, C11D 17/00, C11D 11/00

(54) **Gelförmiges Reinigungsmittel für Spültoiletten**

(30) Priorität: 17.02.1999 DE 19906481
(71) Anmelder: Cognis Deutschland GmbH, 40589 Düsseldorf (DE)
(72) Erfinder: Kitschkel, Ditmar, 40789 Monheim (DE); Stute, Jutta, 50823 Köln (DE); Krohnen, Thomas, 40599 Düsseldorf (DE)

(57) **Zusammenfassung**

Gelförmige Reinigungsmittel für Spültoiletten enthalten nichtionogene Tenside, insbesondere Alkylpolyglykoside sowie kationische Polymere als Verdicker, und weisen vorzugsweise einen pH-Wert unter 7 auf.

## Beschreibung

Die vorliegende Erfindung betrifft gelförmige Reinigungsmittel für Spültoiletten enthaltend nichtionogene Tenside, vorzugsweise Alkylpolyglykoside allein oder in Mischung mit weiteren nichtionischen Tensiden, sowie kationische Polymere als Verdickungsmittel, ein Verfahren zu ihrer Herstellung sowie die Verwendung der kationischen Polymeren als Verdickungsmittel zur Herstellung von gelförmigen Reinigungsmitteln für Spültoiletten.

Als Reinigungsmittel für Spültoiletten werden seit langem Toilettensteine in fester Anbietungsform eingesetzt, die mit Hilfe einer Vorrichtung entweder in den Spülkasten eingehängt oder unter dem Innenrand des WC's befestigt werden. Ihre Aufgabe besteht darin, die Toilette während des Spülvorgangs oberflächlich zu reinigen und insbesondere durch Freisetzung von Duftstoffen unangenehme Gerüche zu überdecken. Insbesondere aufgrund ihrer Aufgabe Duftstoffe freizusetzen, werden Reinigungsmittel für Spültoiletten in der Literatur auch allgemein als Duftspüler bezeichnet. Üblicherweise werden zu ihrer Herstellung Tenside, Buildersubstanzen, anorganische Salze und natürlich Duft- und Farbstoffe eingesetzt. Aus dem Stand der Technik sind eine Vielzahl derartiger Formulierungen bekannt. In der **US 4,534,879** (Procter & Gamble) werden beispielsweise feste Reinigungsmittel beansprucht, die als Tensidkomponente Alkylsulfate mit 9 bis 15 Kohlenstoffatomen, Alkylbenzolsulfonate und anorganische Salze enthalten. Aus der **EP-A 0 014 979** (Henkel) sind Toilettensteine bekannt, die Alkylbenzolsulfonate und Alkylsulfate sowie Fettalkohol- bzw. Alkylphenolethoxylate enthalten. Gegenstand der **DE-C2 43 370 32** (Henkel) sind Toilettensteine mit einem Gehalt an Alkylsulfaten, Alkylethersulfaten und Alkylglukosiden. In der **EP-A 0 268 967** (Henkel) werden Toilettensteine offenbart, die Natriumlaurylsulfat und Fettsäuremonoethanolamid enthalten.

Die beschriebenen Toilettensteine werden in der Regel nach Gieß-, Preß-, Extrudier- oder Granulierverfahren gefertigt, die einen hohen technischen Aufwand erfordern und häufig durch die auftretende Temperaturbelastung (Gieß-/Extrudierverfahren) unerwüschte Parfümverluste erleiden.

Als nachteilig erweist es sich auch, daß die aus ökologischen Gründen verbreiteten Nachfülleinheiten nur nach vollständigem Verbrauch des stückförmigen Körpers eingesetzt werden können. Eine wünschenswerte, beliebige Nachfüllung z.B. zur stärkeren Wirkstofffreisetzung oder insbesondere der intensiveren Duftentfaltung ist nicht möglich.

Aus der deutschen Patentschrift **DE-C-197 15 872** sind gelförmige Toilettenreiniger mit strukturviskosen Eigenschaften bekannt, die den Aufwand der Herstellung erheblich verringern und aufgrund einfacher Technik kostengünstiger zu produzieren sind. Auch das Problem der individuellen Nachfüllmöglichkeit kann durch derartige strukturviskose Wirkstoffzubereitungen gelöst werden. Diese gelförmigen Toilettenreiniger enthalten Polysaccharide, insbesondere Xanthan-Gum, zur Einstellung der strukturviskosen Eigenschaften, und als Tenside zwingend Alkylpolyglykoside sowie ggf. anionische und/oder nichtionische Co-Tenside. Diese gelförmigen Reinigungsmittel müssen jedoch unter Einhaltung besonderer Vorsichtsmaßnahmen bei der Gelbildung hergestellt werden, damit zum einen keine Blasen entstehen und zum anderen die weiteren Inhaltsstoffe in dem Gel gleichmäßig verteilt eingearbeitet werden können.

Aufgabe der vorliegenden Erfindung war es, gelförmige Toilettenreiniger zur Verfügung zu stellen, die zum einen die an sie gestellten Anforderungen hinsichtlich Viskositätsverhalten, Lagerbeständigkeit, gute Reinigungsleistung und ökologische Verträglichkeit erfüllen, sowie zum anderen technisch leichter herstellbar sind.

Zusätzlich sollten die gelförmigen Toilettenreiniger für Spültoiletten ein gutes Anfangsschaumverhalten aufweisen und ein klares Aussehen zeigen. Des weiteren sollten sich die unterschiedlichsten Parfümöle problemlos, insbesondere in Mengen von mindestens 5 Gew.%, einarbeiten lassen.

Ein Gegenstand der vorliegenden Erfindung sind demgemäß gelförmige Reinigungsmittel für Spültoiletten enthaltend Verdicker und nichtionische Tenside sowie ggf. weitere Inhaltsstoffe, **dadurch gekennzeichnet**, daß als Verdicker kationische Polymere enthalten sind.

Als Tenside enthalten die erfindungsgemäßen Mittel zwingend nichtionische Tenside. Nichtionische Tenside im Rahmen der vorliegenden Erfindung können alkoxylierte Alkohole, wie Polyglycolether, Fettalkoholpolygycolether, Alkylphenolpolyglycolether, endruppenverschlossene Polyglycolether, Mischether, Hydroxymischether, alkoxylierte Carbonsäureester, Aminoxide und Alkylpolyglykoside sein. Ebenfalls verwendbar sind Ethylenoxid-Propylenoxid-Blockpolymere und Fettsäurealkanolamide und Fettsäurepolyglycolether. Besonders bevorzugt sind in den erfindungsgemäßen Mitteln als nichtionische Tenside Alkylpolyglykoside alleine oder in Mischung mit weiteren nichtionischen Tensiden enthalten.

Alkylpolyglykoside stellen bekannte nichtionische Tenside dar, die der Formel (**I**) folgen, 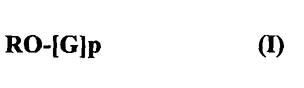 in der R für einen Alkyl- und/oder Alkenylrest mit 6 bis 22 Kohlenstoffatomen, G für einen Zuckerrest mit 5 oder 6 Kohlenstoffatomen und p für eine Zahl zwischen 1 und 10 steht. Sie können nach den einschlägigen Verfahren der präparativen organischen Chemie erhalten werden. Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligo**glucoside**. Die Indexzahl p in der allgemeinen Formel (**I**) gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.

Als weiteres nichtionisches Tensid, vorzugsweise in Mischung mit den beschriebenen Alkylpolyglykosiden, können die erfindungsgemäßen Mittel Fettsäurealkanolamide enthalten, die vorzugsweise der Formel (**II**) folgen, 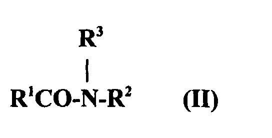 in der R¹CO für einen aliphatischen Acylrest mit 6 bis 22 Kohlenstoffatomen, R² für einen Hydroxyalkylrest mit 2 bis 4 Kohlenstoffatomen und R³ für Wasserstoff oder R² steht. Auch hierbei handelt es sich um bekannte Zusatzstoffe, die gewöhnlich durch Kondensation von Fettsäuren mit Alkanolaminen hergestellt werden. Typische Beispiele sind Kondensationsprodukte von Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure bzw. deren technischen Mischungen mit Monoethanolamin und Diethanolamin. Vorzugsweise werden Fettsäurealkanolamide der Formel (**II**) eingesetzt, in der R¹CO für einen Acylrest mit 12 bis 18 Kohlenstoffatomen, R² für einen Hydroxyethylrest und R³ für R² oder Wasserstoff steht. Besonders bevorzugt ist der Einsatz von C_{12/14}- bzw. C_{12/18}-Kokosfettsäuremono- bzw. - diethanolamid.

Als weitere Gruppe nichtionischer Tenside kommen schließlich Alkoholethoxylate in Frage, die vorzugsweise der Formel (**III**) folgen, 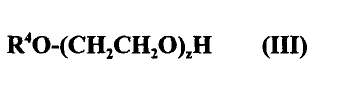 in der R⁴ für einen linearen oder verzweigten Alkyl- und/oder Alkenylrest mit 12 bis 18 Kohlenstoffatomen und z für Zahlen von 1 bis 50, vorzugsweise 5 bis 30 steht. Auch diese Stoffe stellen bekannte großtechnische Produkte dar, die für gewöhnlich durch basenkatalysierte Anlagerung von Ethylenoxid an primäre Alkohole hergestellt werden. In Abhängigkeit der verwendeten Katalysatoren (z.B. Natriummethylat oder calciniertes Hydrotalcit) können die Ethoxylate eine konventionelle oder eingeengte Homologenverteilung aufweisen. Bei den Alkoholethoxylaten kann es sich um Addukte von 1 bis 50 Mol Ethylenoxid an Fettalkohole ("Fettalkoholethoxylate") oder Oxoalkohole ("Oxoalkoholethoxylate") handeln. Typische Beispiele sind die Ethoxylate von Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Bevorzugt sind Addukte von 1 bis 50, vorzugsweise 5 bis 30 und insbesondere 10 bis 20 Mol Ethylenoxid an technische Fettalkohole mit 12 bis 18 Kohlenstoffatomen, wie beispielsweise Kokos-, Palm-, Palmkern- oder Talgfettalkohol.

Als weitere Gruppe geeigneter nichtionischer Tenside, vorzugsweise in Mischung mit den schon beschriebenen Alkylpolyglykosiden, können alkoxylierte Carbonsäureester der Formel (**IV**), enthalten sein 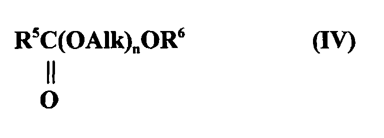 in der R⁵CO für einen aliphatischen Acylrest, AlkO für CH₂CH₂O, CHCH₃CH₂O und/oder CH₂CHCH₃O, n für Zahlen von 1 bis 20 und R⁶ für einen aliphatischen Alkylrest steht.

Alkoxylierte Carbonsäureester der Formel (**IV**) sind aus dem Stand der Technik bekannt. So sind beispielsweise derartige alkoxylierte Carbonsäureester durch Veresterung von alkoxylierten Carbonsäuren mit Alkoholen zugänglich. Bevorzugt im Sinne der vorliegenden Erfindung werden die Verbindungen jedoch durch Umsetzung von Carbonsäureestern mit Alkylenoxiden unter Verwendung von Katalysatoren hergestellt, insbesondere unter Verwendung von calciniertem Hydrotalcit gemäß der deutschen Offenlegungsschrift **DE-A- 39 14 131**, die Verbindungen mit einer eingeschränkten Homologenverteilung liefern. Bevorzugt gemäß der vorliegenden Erfindung werden alkoxylierte Carbonsäureester der allgemeinen Formel (**IV**), in der R⁵CO für einen aliphatischen Acylrest mit 6 bis 22 Kohlenstoffatomen, AlkO für einen CH₂CH₂O-, CHCH₃CH₂O- und/oder CH₂-CHCH₃O-Rest, n durchschnittlich für Zahlen von 3 bis 20 und R⁶ für einen aliphatischen Alkylrest mit 1 bis 22 Kohlenstoffatomen steht.

Bevorzugte Acylreste leiten sich von Carbonsäuren mit 6 bis 22 Kohlenstoffatomen natürlicher oder synthetischer Herkunft ab, insbesondere von geradkettigen gesättigten und/oder ungesättigten Fettsäuren einschließlich technischer Gemische derselben, wie sie durch Fettspaltung aus tierischen und/oder pflanzlichen Fetten und Ölen zugänglich sind, zum Beispiel aus Kokosöl, Palmkernöl, Palmöl, Sojaöl, Sonnenblumenöl, Rüböl, Baumwollsaatöl, Fischöl, Rindertalg und Schweineschmalz. Beispiele für derartige Carbonsäuren sind Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Olsäure, Elaidinsäure, Petroselinsäure, Linolsäure, Linolensäure, Elaeostearinsäure, Arachinsäure, Gadoleinsäure, Behensäure und/oder Erucasäure. Insbesondere steht R⁵CO für einen geradkettigen, geradzahligen Acylrest mit 8 bis 18 Kohlenstoffatomen. Bevorzugte Alkylreste R⁶ leiten sich von primären, aliphatischen monofunktionellen Alkoholen mit 1 bis 22 Kohlenstoffatomen ab, die gesättigt und/oder ungesättigt sein können. Beispiele für geeignete Monoalkohole sind Methanol, Ethanol, Propanol, Butanol, Pentanol sowie die Hydrierungsprodukte der oben genannten Carbonsäuren mit 6 bis 22 Kohlenstoffatomen. Insbesondere steht R⁶ für einen Methylrest. Vorzugsweise steht AlkO für einen CH₂CH₂O-Rest. Insbesondere geeignet sind alkoxylierte Carbonsäureester der Formel (**IV**), in der R⁵CO für einen geradkettigen, geradzahligen Acylrest mit 8 bis 18 Kohlenstoffatomen, AlkO für einen CH₂CH₂O-Rest, n durchschnittlich für Zahlen von 5 bis 15 und R⁶ für einen Methylrest steht. Beispiele für derartige Verbindungen sind mit im Durchschnitt 5, 7, 9 oder 11 Mol Ethylenoxid alkoxylierte Carbonsäuremethylester.

Besonders bevorzugt enthalten die erfindungsgemäßen Mittel als nichtionisches Tensid Aminoxide, vorzugsweise in Mischung mit den schon beschriebenen Alkylpolygylkosiden. Zur Herstellung von Alkylaminoxiden geht man von tertiären Fettaminen aus, die üblicherweise entweder einen langen und zwei kurze oder zwei lange und einen kurzen Alkylrest aufweisen, und oxidiert sie in Gegenwart von Wasserstoffperoxid. Die im Sinne der Erfindung in Betracht kommenden Alkylaminoxide folgen der Formel (**V**), 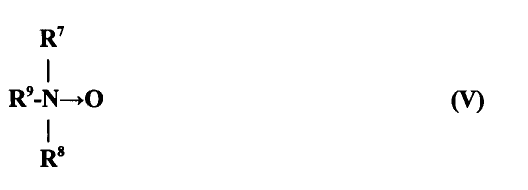 in der R⁹ für einen linearen oder verzweigten Alkylrest mit 12 bis 18 Kohlenstoffatomen sowie R⁷ und R⁸ unabhängig voneinander für R⁹ oder einen gegebenenfalls hydroxysubstituierten Alkylrest mit 1 bis 4 Kohlenstoffatomen stehen. Vorzugsweise werden Aminoxide der Formel (**V**) eingesetzt, in der R⁹ und R⁷ für C_{12/14}- bzw. C_{12/18}-Kokosalkylreste stehen und R⁸ einen Methyl- oder einen Hydroxyethylrest bedeutet. Ebenfalls bevorzugt sind Aminoxide der Formel (**V**), in denen R⁹ für einen C_{12/14}- bzw. C_{12/18}- Kokosalkylrest steht und R⁷ und R⁸ die Bedeutung eines Methyl- oder Hydroxyethylrestes haben.

Innerhalb der Gruppe der Aminoxide kommen auch die sogenannten Amidoaminoxide in Betracht, die der allgemeinen Formel (**VI**) folgen, 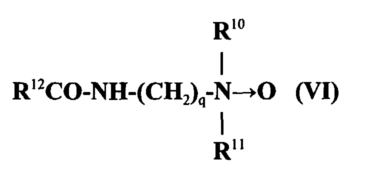 in der R¹²CO für einen aliphatischen Acylrest mit 6 bis 22 Kohlenstoffatomen und 0 oder 1 bis 3 Doppelbindungen, q für Zahlen von 1 bis 3 steht und R¹⁰, R¹¹ unabhängig voneinander für R⁹ oder einen gegebenenfalls hydroxysubstituierten Alkylrest mit 1 bis 4 Kohlenstoffatomen stehen. Ein typisches Beispiel ist Dimethyl-N-(kokosamidopropyl)aminoxid.

Die erfindungsgemäßen Mittel können die nichtionischen Tenside, vorzugsweise die Alkylpolyglykoside alleine oder in Mischung mit den Aminoxiden, als alleiniges Tensid enthalten. Es ist aber auch möglich, daß die erfindungsgemäßen Mittel zusätzlich anionische und vorzugsweise kationische und/oder amphotere bzw. zwitterionische Tenside enthalten. In geringen Mengen können anionische Tenside, beispielsweise aliphatische Sulfate wie Fettalkoholsulfate, Fettalkoholethersulfate, Fettsäurepolyglykolestersulfate, Dialkylethersulfate, Monoglyceridsulfate und aliphatische Sulfonate wie Alkansulfonate, Olefinsulfonate, Ethersulfonate, n-Alkylethersulfonate, Estersulfonate, und Lingninsulfonate enthalten sein. Als anionische Tenside werden bevorzugt Fettalkoholsulfate, Fettalkoholethersulfate und/oder Fettsäurepolyglykolestersulfate. Da die anionischen Tenside jedoch mit den kationischen Polymeren als Verdickungsmittel in Wechselwirkung treten, kann es insbesondere bei starken anionischen Tensiden zu Ausfällungen kommen, vor allen Dingen auch dann, wenn die Menge an anionischen Tensiden über 10 Gew.% - bezogen auf Gesamttensidmenge - beträgt. Um klare Mittel zu gewährleisten, ist es im Sinne der vorliegenden Erfindung besonders bevorzugt, wenn die erfindungsgemäßen Mittel keine anionischen Tenside enthalten.

Als kationische Tenside können die erfindungsgemäßen Mittel quartäre Ammoniumverbindungen und Esterquats, insbesondere quaternierte Fettsäuretrialkanolaminester-Salze enthalten.

Unter der Bezeichnung "Esterquats" werden im allgemeinen quaternierte Fettsäuretriethanolaminestersalze verstanden. Es handelt sich dabei um bekannte Stoffe, die man nach den einschlägigen Methoden der präparativen organischen Chemie erhalten kann. In diesem Zusammenhang sei auf die internationale Patentanmeldung **WO 91/01295** (Henkel) verwiesen, nach der man Triethanolamin in Gegenwart von unterphosphoriger Säure mit Fettsäuren partiell verestert, Luft durchleitet und anschließend mit Dimethylsulfat oder Ethylenoxid quaterniert. Aus der deutschen Patentschrift **DE 43 087 94 C1** (Henkel) ist überdies ein Verfahren zur Herstellung fester Esterquats bekannt, bei dem man die Quaternierung von Triethanolaminestern in Gegenwart von geeigneten Dispergatoren, vorzugsweise Fettalkoholen, durchführt. Übersichten zu diesem Thema sind beispielsweise von R.Puchta et al. in **Tens.Surf.Det., 30, 186 (1993)**, M.Brock in **Tens.Surf.Det. 30, 394 (1993)**, R.Lagerman et al. in **J.Am.Oil.Chem.Soc., 71, 97 (1994)** sowie I.Shapiro in **Cosm.Toil. 109, 77 (1994)** erschienen.

Einer Ausführungsform der vorliegenden Erfindung entsprechend enthalten die erfindungsgemäßen Mittel neben den nichtionischen Tensiden amphotere bzw. zwitterionische Tenside. Typische Beispiele für amphotere bzw. zwitterionische Tenside sind Alkylbetaine, Alkylamidobetaine, Aminopropionate, Imidazoliniumbetaine und Sulfobetaine.

Betaine stellen bekannte Tenside dar, die überwiegend durch Carboxyalkylierung, vorzugsweise Carboxymethylierung von aminischen Verbindungen hergestellt werden. Vorzugsweise werden die Ausgangsstoffe mit Halogencarbonsäuren oder deren Salzen, insbesondere mit Natriumchloracetat kondensiert, wobei pro Mol Betain ein Mol Salz gebildet wird. Ferner ist auch die Anlagerung von ungesättigten Carbonsäuren, wie beispielsweise Acrylsäure möglich. Zur Nomenklatur und insbesondere zur Unterscheidung zwischen Betainen und "echten" Amphotensiden sei auf den Beitrag von U.Ploog in **Seifen-Öle-Fette-Wachse, 108, 373 (1982)** verwiesen. Beispiele für geeignete Betaine stellen die Carboxyalkylierungsprodukte von sekundären und insbesondere tertiären Aminen dar, die der Formel (**VII**) folgen, 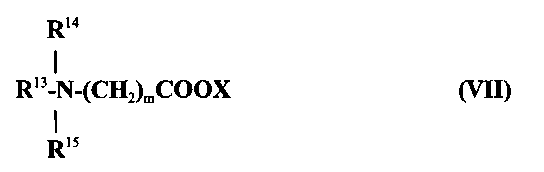 in der R¹³ für Alkyl- und/oder Alkenylreste mit 6 bis 22 Kohlenstoffatomen, R¹⁴ für Wasserstoff oder Alkylreste mit 1 bis 4 Kohlenstoffatomen, R¹⁵ für Alkylreste mit 1 bis 4 Kohlenstoffatomen, m für Zahlen von 1 bis 6 und X für ein Alkali- und/oder Erdalkalimetall oder Ammonium steht. Typische Beispiele sind die Carboxymethylierungsprodukte von Hexylmethylamin, Hexyldimethylamin, Octyldimethylamin, Decyldimethylamin, Dodecylmethylamin, Dodecyldimethylamin, Dodecylethylmethylamin, C_{12/14}-Kokosalkyldimethylamin, Myristyldimethylamin, Cetyldimethylamin, Stearyldimethylamin, Stearylethylmethylamin, Oleyldimethylamin, C_{16/18}-Talgalkyldimethylamin sowie deren technische Gemische. Weiterhin kommen auch Carboxyalkylierungsprodukte von **Amidoaminen** in Betracht, die sogenannten Glycinate, die der Formel (**VIII**) folgen, 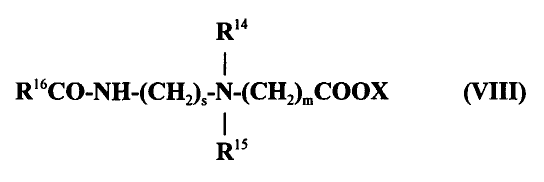 in der R¹⁶CO für einen aliphatischen Acylrest mit 6 bis 22 Kohlenstoffatomen und 0 oder 1 bis 3 Doppelbindungen, s für Zahlen von 1 bis 3 steht und R¹⁴, R¹⁵, m und X die oben angegebenen Bedeutungen der Formel (VII) haben. Typische Beispiele sind Umsetzungsprodukte von Fettsäuren mit 6 bis 22 Kohlenstoffatomen, namentlich Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Gemische, mit N,N-Dimethylaminoethylamin, N,N-Dimethylaminopropylamin, N,N-Diethylaminoethylamin und N,N-Diethylaminopropylamin, die mit Natriumchloracetat kondensiert werden. Bevorzugt ist der Einsatz eines Kondensationsproduktes von C_{8/18}-Kokosfettsäure-N,N-dimethylaminopropylamid mit Natriumchloracetat. In einer bevorzugten Ausführungsform der Erfindung, sind als nichtionisches Tensid Alkylpolyglykoside alleine oder in Mischung mit den Aminoxiden enthalten. Sofern die Alkylpolyglykoside das alleinige nichtionische Tensid sind, ist es im Rahmen einer weiteren Ausführungsform bevorzugt, diese mit amphoteren bzw. zwitterionischen Tensiden, insbesondere aus der Gruppe der Amidoaminen zu mischen.

Demgemäß enthalten die erfindungsgemäßen Mittel vorzugsweise Tensidgemische aus
(a) 25 bis 100, vorzugsweise 50 bis 100 Gew.-% Alkylpolyglykoside,
(b) 0 bis 75, vorzugsweise 15 bis 50 Gew.-% Aminoxide und
(c) 0 bis 75, vorzugsweise 15 bis 50 Gew.-% amphotere bzw. zwitterionische Tenside
mit der Maßgabe, daß sich die Mengen zu 100 Gew.-% - bezogen auf Tensidmischung - ergänzen.

Die erfindungsgemäßen Mittel enthalten die beschriebenen Tensidmischungen in Mengen von 1 bis 65, vorzugsweise von 3 bis 30 Gew.%.

Die erfindungsgemäßen gelförmigen Mittel enthalten außer den schon beschriebenen Tensiden zusätzlich kationische Polymere als Verdicker. Dabei sollen die kationischen Polymere als Verdicker in der Lage sein, eine Strukturviskosität bei dem erfindungsgemäßen gelförmigen Mittel einzustellen. Bevorzugt sollen die kationischen Polymere ermöglichen, daß die erfindungsgemäßen Mittel strukturviskos sind und eine Fließgrenze aufweisen, d.h. ohne äußere Kräfteeinwirkung (im Ruhezustand) sind die Mittel praktisch nicht fließend und verhalten sich wie ein Festkörper, beim Drücken der Mittel (äußere Kräfteeinwirkung) werden die Mittel fließfähig und können ohne Probleme in die Spülkörbchen eingefüllt werden. Vorzugsweise liegt die Viskosität der Mittel im Bereich von 5 000 bis 50 000 mPas, gemessen mit dem Brookfield Rotationsviskosimeter, Typ RVT mit der Spindel 6 bei 20 U/min und bei 22 °C.
Die genannten Eigenschaften können mit dem kationischen Polymer Polygel K 100 ^{R} der 3V Sigma S.P.A. hervorragend erreicht werden.

Vorzugsweise sind die kationischen Polymere in Mengen von 1,0 bis 10,0 Gew.%, insbesondere von 1,5 bis 6 Gew.%, - bezogen auf Mittel - enthalten.

Falls die erfindungsgemäßen Mittel in ihrem Viskositätsverhalten modifiziert werden sollen, können neben den kationischen Polymeren übliche Verdickungsmittel, beispielsweise Harnstoff, Natriumchlorid, Natriumsulfat, Magnesiumsulfat, Ammoniumchlorid und Magnesiumchlorid sowie die Kombination dieser Verdickungsmittel in den erfindungsgemäßen Mitteln enthalten sein. Eine weitere Möglichkeit das Viskositätsverhalten der Mittel zu erhöhen, besteht darin, den pH-Wert der Mittel auf Werte unter 7, vorzugsweise unter 5 durch Zugabe von Säuren, beispielsweise von Citronen-, Ameisen-, Milch- oder Essigsäure, einzustellen. Durch die Säurezugabe, insbesondere von Ameisensäure, wird eine höhere Endviskosität der Mittel erreicht, die im angegebenen Viskositätsbereich liegt.

Die erfindungsgemäßen gelförmigen Reinigungsmittel können außer den schon beschriebenen Tensiden und den Verdickern kalklösende Mittel, Builder, Parfüme, Lösungsmittel, Parfümsolubilisatoren, Konservierungsmittel, Farbstoffe sowie keimhemmende Mittel enthalten.

Als kalklösende Mittel sind vorzugsweise kalklösende Säuren wie die Zitronensäure, Ameisensäure, Essigsäure, Milchsäure oder deren wasserlöslichen Salze enthalten, bevorzugt in einer Menge von 0,5 bis 12 Gew.-%, besonders bevorzugt von 2 bis 7 Gew.-% - bezogen auf Mittel -.

Die vorzugsweise wasserlöslichen Farbstoffe sind entweder für die Farbgebung des Mittels oder für die Farbgebung der den Behälter umspielenden Flüssigkeit enthalten. Bevorzugt liegt der Gehalt an wasserlöslichen Farbstoffen unter 1 Gew.-% und dient zur Verbesserung der Optik des Produktes. Wenn ein zusätzliches Farbsignal beim Einspülvorgang gewünscht ist, kann der Gehalt an wasserlöslichen Farbstoffen bis 5 Gew.-% betragen.

Die hygienische Wirkung kann durch Zusatz keimhemmender Mittel verstärkt werden. Geeignete keimhemmende Mittel sind insbesondere Isothiazolingemische, Natriumbenzoat und/oder Salicylsäure. Die Menge dieser keimhemmenden Mittel hängt stark von der Wirksamkeit der jeweiligen Verbindung ab und kann bis zu 5 Gew.-% befragen. Vorzugsweise sind die keimhemmenden Mittel in Mengen von 0,01 Gew.-% bis 3 Gew.-% enthalten.

Als Lösungsmittel, insbesondere für Farbstoffe und Parfümöle, können in den erfindungsgemäßen Mitteln beispielsweise Alkanolamine, Polyole wie Ethylenglycol, Propylenglycol, 1,2 Glycerin und andere ein- und mehrwertige Alkohole, sowie Alkylbenzolsulfonate mit 1 bis 3 Kohlenstoffatomen im Alkylrest enthalten sein. Besonders bevorzugt ist dabei die Gruppe der niederen Alkohole, ganz besonders Ethanol. Der Gehalt der Lösungsmittel ist abhängig von der Art und Menge der zu lösenden Bestandteile und liegt in der Regel zwischen 0 und 10, vorzugsweise zwischen 0,01 und 7 Gew.%.

Als Parfümsolubilisatoren können in den erfindungsgemäßen Mitteln Polyolfettsäureester, beispielsweise mit 7 Mol Ethylenoxid alkoxyliertes Glycerin, welches mit Kokosfettsäure verestert ist (Cetiol HE ^{R} der Henkel KGaA) und/oder mit 40 oder 60 Mol Ethylenoxid alkoxyliertes gehärtetes Ricinusöl (Eumulgin HRE 40 bzw. 60 ^{R}; der Henkel KGaA) und/oder 2-Hydroxyfettalkoholethoxylate (Eumulgin L ^{R}; der Henkel KGaA) enthalten sein. Die Menge der Parfümsolubilisatoren in den erfindungsgemäßen Mitteln liegt in der Regel zwischen 0 und 10, vorzugsweise zwischen 1 und 7 Gew.%.

Weitere fakultative Bestandteile der erfindungsgemäßen Mittel sind Builder, vorzugsweise wasserlösliche Builder, da sie auf harten Oberflächen in der Regel weniger dazu tendieren unlösliche Rückstände zu bilden. Übliche Builder, die im Rahmen der Erfindung zugegen sein können, sind die niedermolekularen Polycarbonsäuren und ihre Salze, die homopolymeren und copolymeren Polycarbonsäuren und ihre Salze, die Citronensäure und ihre Salze, die Carbonate, Phosphate und Silikate. Zu wasserunlöslichen Buildern zählen die Zeolithe, die ebenfalls verwendet werden können, ebenso wie Mischungen der vorgenannten Buildersubstanzen. Besonders bevorzugt ist die Gruppe der Citrate. Die Builder können in Mengen von 0 bis 5 Gew.% in den erfindungsgemäßen Mitteln enthalten sein.

Bei den fakultativ enthaltenen Parfümen handelt es sich um die aus dem Stand der Technik gängigen. Die Menge der Dosierung ist abhängig von der gewünschten Duftintensität und liegt im Bereich von 0 bis 15 Gew.%, vorzugsweise von 2 bis 12, bevorzugt von 3 -10 und insbesondere von 5 bis 7 Gew.%.

Des weiteren können noch übliche Konservierungsmittel in den üblichen Mengen von 0 bis 1 Gew.% enthalten sein.

Der zu 100 Gew.% fehlende Rest der gelförmigen Reinigungsmittel ist Wasser.

Ein weiterer Gegenstand der vorliegenden Erfindung betrifft die Herstellung von gelförmigen Reinigungsmitteln für Spültoiletten nach Anspruch 1, dadurch gekennzeichnet, daß eine Dispersion aus kationischen Polymeren, nichtionischem Tensid sowie ggf. weiteren Inhaltsstoffen in Wasser hergestellt und ggf. anschließend durch Säurezugabe ein pH-Wert unter 7 eingestellt wird.

Nach dem erfindungsgemäßen Verfahren ist es wesentlich, daß die fakultative pH-Wert-Einstellung auf Werte unter 7 erst dann erfolgt, wenn alle Inhaltsstoffe der erfindungsgemäßen Mittel in Wasser verteilt vorliegen. Bei Säurezugabe und damit bei pH-Werteinstellung unter 7 erfolgt eine weitere Viskositätszunahme des Gels. Nach der Gelbildung ist es dann sehr schwierig, weitere Inhaltsstoffe in die Mittel gleichmäßig einzubringen. Im Sinne des erfindungsgemäßen Verfahrens können sowohl zu vorgelegtem Wasser und ggf. vorhandenen Lösungsvermittlern erst die Tenside und weitere Bestandteile und dann die kationischen Polymere oder erst die kationischen Polymere und dann weitere Inhaltsstoffe zugegeben werden. Bevorzugt wird nach der ersten Variante gearbeitet, da nach Zugabe der kationischen Polymere die Rührgeschwindigkeit nur noch mittel bis moderat sein sollte.

Nach dem erfindungsgemäßen Verfahren werden Mittel erhalten, die keine nennenswerten Blasen aufweisen und zudem optimal verteilt alle Inhaltsstoffe der Mittel enthalten. Falls Blasen für die Optik gewünscht werden, können diese selbstverständlich erzeugt werden.

Ein weiterer Gegenstand der vorliegenden Erfindung betrifft die Verwendung von kationischen Polymeren als Verdicker zur Herstellung von gelförmigen Reinigungsmitteln für Spültoiletten.

Im Sinne der Erfindung sollen die gelförmigen Reinigungsmittel als Toilettengele in dafür vorgesehene Behälter beispielsweise am Innenrand des WC's aufbewahrt werden. Es handelt sich somit um stationär angebrachte gelförmige Reinigungsmittel, insbesondere für Spültoiletten. Die erfindungsgemäßen Mittel zeichnen sich durch ein gutes Anfangschäumverhalten aus, das durch Zusatz von Aminoxiden oder amphoteren bzw. zwitterionischen Tensiden noch zusätzlich zu steigern ist. Weiterhin zeichnen sich die erfindungsgemäßen Mittel durch eine erhöhte Lebensdauer aus, d.h. eine Erhöhung der Abspülzahl wird erreicht. Ein besonderer Vorteil ergibt sich auch dadurch, daß die kationischen Polymere neben ihrer Verdickerwirkung selber ein gewisses Parfümöltragevermögen aufweisen, wodurch die Menge an einzuarbeitendem Parfüm gesteigert werden kann. Eine hohe Menge an eingearbeitetem Parfüm wird auch deshalb gewünscht, da die Mittel neben der Reinigungswirkung als Duftspender wirken sollen. Sofern Parfümmengen über 4 Gew.-% eingearbeitet werden sollen, hat es sich als vorteilhaft erwiesen, wenn der pH-Wert der Mittel nicht durch Säurezugabe auf Werte unter 7 eingestellt wird, da es ansonsten leicht zu Trübungen kommen kann.

### Beispiele

### Allgemeine Herstellvorschrift:

Es wurde die berechnete Menge Wasser vorgelegt und alle Bestandteile der Mittel in den in Tabelle 1 angegebenen Mengen an Tensid, Parfüm, und Ethanol zugegeben und untergerührt. Anschließend wurde die in Tabelle 1 wiedergegebene Menge an kationischem Polymer zugegeben und unter moderaten Bedingungen untergerührt. Nachdem sich alles gut verteilt hatte, wurde ggf. die in Tabelle 1 angegebene Menge an Ameisensäure eingerührt. Man erhielt gelförmige Produkte. Das Mittel 13 war leicht trübe.

**Tabelle 1**

| Angaben in % Aktivsubstanz | | | | | | | | | | | | | |
|---|---|---|---|---|---|---|---|---|---|---|---|---|---|
| | **1** | **2** | **3** | **4** | **5** | **6** | **7** | **8** | **9** | **10** | **11** | **12** | **13** |
| C8/10-APG ¹ | 10 | 10 | 5 | 4 | 8 | 4 | 8 | 4 | 8 | 4 | 16 | 16 | 16 |
| C12/14 APG ² | | | 5 | 4 | | 4 | | 4 | | 4 | - | - | - |
| Ethanol | 3 | 3 | 4 | 4 | 4 | 4 | 4 | 4 | 4 | 4 | 3 | 3 | 3 |
| Ameisensäure | 0,5 | 0,5 | 0,5 | 0,5 | 0,5 | 0,5 | 0,5 | 0,5 | 0,5 | 0,5 | 0,5 | 0 | 0,5 |
| Polygel K100 | 3 | 3 | 4 | 4 | 4 | 4 | 4 | 4 | 4 | 4 | 3 | 3 | 3 |
| Parfüm 50-4149 | 3 | 5 | 5 | 5 | 5 | 5 | 5 | 5 | 5 | 5 | 3 | 5 | 5 |
| Betain ³ | | | | 2 | 8 | | | | | 8 | | - | |
| Amidoaminoxid⁴ | | | | | | 2 | 8 | | | | | - | |
| Alkylaminoxid⁵ | | | | | | | | 2 | 8 | | | | |
| Eumulgin HRE 40 | | | | | | | | | | | - | 2 | 2 |
| Wasser | Zu 100 | Zu 100 | Zu 100 | Zu 100 | Zu 100 | Zu 100 | Zu 100 | Zu 100 | Zu 100 | Zu 100 | Zu 100 | Zu 100 | Zu 100 |

| | | | | | | | | | | | | | |
|---|---|---|---|---|---|---|---|---|---|---|---|---|---|
| Legende 1 = Alkylpolyglucosid mit 8 und 10 C-Atomen im Alkylrest; DP= 1,6; Glucopon 220 ^{R}; Henkel KGaA | | | | | | | | | | | | | |
| 2 = Alklypolyglucosid mit 12 und 14 C-Atomen (70:30) im Alkylrest; DP = 1,4; Glucopon 600 ^{R}; Henkel KGaA | | | | | | | | | | | | | |
| 3 = Dimethyl-N-Kokosalkylammoniumbetain | | | | | | | | | | | | | |
| 4 = Dimethyl-N-(Kokosamidopropyl)aminoxid | | | | | | | | | | | | | |
| 5 = Dimethyl-N(C12/14alkyl)aminoxid | | | | | | | | | | | | | |

## Patentansprüche

1. Gelförmige Reinigungsmittel für Spültoiletten enthaltend Verdicker und nichtionische Tenside sowie ggf. weitere Inhaltsstoffe, **dadurch gekennzeichnet**, daß als Verdicker kationische Polymere enthalten sind.

2. Gelförmige Reinigungsmittel nach Anspruch 1, **dadurch gekennzeichnet**, daß als nichtionisches Tensid Alkylpolyglykoside enthalten sind.

3. Gelförmige Reinigungsmittel nach einem der Ansprüche 1 oder 2, **dadurch gekennzeichnet**, daß als nichtionisches Tensid Aminoxide enthalten sind.

4. Gelförmige Reinigungsmittel nach einem der Ansprüche 1 bis 3, **dadurch gekennzeichnet**, daß zusätzlich amphotere bzw. zwitterionische Tenside enthalten sind, vorzugsweise Betaine.

5. Gelförmige Reinigungsmittel nach einem der Ansprüche 1 bis 4, **dadurch gekennzeichnet**, daß Tensidgemische enthalten sind aus
(a) 25 bis 100, vorzugsweise 50 bis 100 Gew.-% Alkylpolyglykoside,
(b) 0 bis 75, vorzugsweise 15 bis 50 Gew.-% Aminoxide und
(c) 0 bis 75, vorzugsweise 15 bis 50 Gew.-% amphotere bzw. zwitterionische Tenside
mit der Maßgabe, daß sich die Mengen zu 100 Gew.-% - bezogen auf Tensidmischung ergänzen.

6. Gelförmige Reinigungsmittel nach einem der Ansprüche 1 bis 5, **dadurch gekennzeichnet**, daß sie als Verdicker das kationische Polymer Polygel K 100 ^{R} der 3 V Sigma enthalten.

7. Gelförmiges Reinigungsmittel nach einem der Ansprüche 1 bis 6, **dadurch gekennzeichnet**, daß die Mittel eine Viskosität von 5 000 bis 50 000 mPas aufweisen.

8. Gelförmige Reinigungsmittel nach einem der Ansprüche 1 bis 7, **dadurch gekennzeichnet**, daß sie einen pH-Wert unter 7 aufweisen.

9. Verfahren zur Herstellung von gelförmigen Reinigungsmitteln für Spültoiletten nach Anspruch 1, **dadurch gekennzeichnet**, daß eine Dispersion aus kationischen Polymeren, nichtionischem Tensid sowie ggf. weiteren Inhaltsstoffen in Wasser hergestellt und ggf. anschließend durch Säurezugabe ein pH-Wert unter 7 eingestellt wird.

10. Verwendung von kationischen Polymeren als Verdicker zur Herstellung von gelförmigen Reinigungsmitteln für Spültoiletten.
